# EUROPÄISCHE PATENTSCHRIFT

(11) **EP 1 180 362 B1**
(45) Veröffentlichungstag und Bekanntmachung des Hinweises auf die Patenterteilung: **13.02.2008**
(21) Anmeldenummer: 01114090.2
(22) Anmeldetag: 09.06.2001
(51) Int. Cl.: A61K 8/81, A61Q 19/10, C11D 3/37, C11D 3/43

(54) **Mittel zur Reinigung der Haut**
Cleansing compositions for the skin
Compositions de nettoyage de la peau

(30) Priorität: 18.08.2000 DE 10040884
(43) Veröffentlichungstag der Anmeldung: 20.02.2002
(73) Patentinhaber: Stockhausen GmbH, 47805 Krefeld (DE)
(72) Erfinder: Veeger, Marcel, 47574 Goch (DE); Klotz, Andreas, 41516 Grevenbroich (DE); Nauels, Bernd, 47906 Kempen (DE)
(74) Vertreter: Wolff, Felix

(56) Entgegenhaltungen:
- US-A- 3 734 686
- US-A- 3 862 906
- US-A- 4 655 957
- US-A- 4 968 447
- US-A- 5 538 663
- US-A- 5 830 843
- BRIDGE B.; FOLKES M.J.; WOOD B.R.: 'Investigation into the dc conductivity of colloidally dispersed gold/polymer composites' JOURNAL OF PHYSICS D: APPLIED PHYSICS Bd. 23, Nr. 13, 14 Juli 1990, BRISTOL, UK, Seiten 890 - 898

## Beschreibung

Die vorliegende Erfindung betrifft ein Mittel zur Reinigung der Haut, welches dazu geeignet ist, auf der Haut verrieben zu werden, um Partikel auszubilden, die bei weiterem Reiben von der Haut abfallen, und die Haut ohne Zusatz von Wasser zu reinigen, enthaltend eine flüssige natürliche und/oder synthetische wasserbasierende Polyisoprenlatexemulsion mit einem Durchmesser der Polymerpartikel von 0,05 bis 5 µm, ausschließlich mindestens ein organisches Lösemittel, das eine geringere Flüchtigkeit als Ethanol und einen Siedepunkt > 78,32 °C aufweist, und Wasser.

Mittel zur Reinigung der Haut, insbesondere sogenannte Handwaschpasten, bei denen die Entfernung des Schmutzes ohne den Zusatz von Wasser erfolgt, sind seit längerem bekannt. Da bei diesen Mitteln die Haut nach der Reinigung auch nicht mit Wasser gespült werden muß, kann auf den Einsatz eines Trocknungstuches verzichtet werden.

Aus der DD 272 099 ist eine Waschpaste zur Reinigung öl- und fettverschmutzter Hände bekannt, bei der auf die Verwendung von Wasser verzichtet werden kann. Die Handwaschpaste beinhaltet 1-3% eines wässrigen Copolymerisatlatex aus Styrol, 1, 3-Butadien, Methacrylsäure und einem Veresterungsprodukt der Diels-Alder-Addukte der Maleinsäure.

In der WO 00/17300 wird ein Hautreinigungsmittel gelehrt, das aus einer Latexemulsion, einer Organsiliconverbindung, Emollients, oberflächenaktiven Substanzen und Wasser besteht. Die Latexemulsion weist bevorzugt ein StyrolButadien Copolymer auf.

Die oben genannten Hautreinigungsmittel haben den Nachteil, daß sie nur bedingt hautverträglich, toxikologisch nicht einwandfrei und/oder umweltgefährdend sind.

Es stellt sich deshalb die Aufgabe, ein Hautreinigungsmittel zur Verfügung zu stellen, daß toxikologisch unbedenklich, hautverträglich und für die Umwelt weniger belastend ist, ohne daß die Reinigungsleistung negativ beeinflußt wird.

Die Aufgabe wird erfindungsgemäß durch ein Mittel zur Reinigung der Haut gelöst, welches dazu geeignet ist, auf der Haut verrieben zu werden, um Partikel auszubilden, die bei weiterem Reiben von der Haut abfallen, und die Haut ohne Zusatz von Wasser zu reinigen, das eine flüssige natürliche und/oder synthetische wasserbasierende Latexemulsion mit einem Durchmesser der Polymerpartikel von 0,05 bis 5 µm, ausschließlich mindestens ein organisches Lösemittel, das eine geringere Flüchtigkeit als Ethanol und einen Siedepunkt > 78,32 °C aufweist und Wasser enthält.

Erfindungsgemäß enthält das Mittel zur Reinigung der Haut eine flüssige natürliche und/oder synthetische wasserbasierende Polyisoprenlatexemulsion. Polyisoprenlatexemulsionen im Sinne der Erfindung sind Dispersionen von fein verteilten natürlichen und/oder synthetischem Polyisopren in einem im wesentlichen wässrigen Dispersionsmittel. Die Polymere umfassen Natur- und Synthesekautschuk. Der Durchmesser der Polymerpartikel beträgt 0,05 bis 5 µm.

In einer bevorzugten Ausführungsform enthält das erfindungsgemäße Mittel zur Reinigung der Haut:
a. 10-80 Gew.-% einer flüssigen natürlichen und/oder synthetischen wasserbasierenden Polyisoprenlatexemulison,
b. 1-15 Gew.-% eines organischen Lösemittels, das eine geringere Flüchtigkeit als Ethanol und einen Siedepunkt > 78,32 °C aufweist,
c. 0-10 Gew.-% mindestens eines Tensides, vorzugsweise mindestens eines Fettalkoholethoxylates, Fettalkoholethersulfates, Succinates, Sarkosides und/oder Glucosides,
d. 0-10 Gew.-% Abrasiva, vorzugsweise gebleichtes Walnußschalenmehl und/oder nicht quellbare Stärke,
e. 0-0,1 Gew.-% Verdickungsmittel,
f. ggf. kosmetische Hilfs- und Zusatzstoffe und/oder Wirkstoffe,
g. 10-60 Gew.-% Wasser,
wobei die Summe 100 Gew.% ergibt.

Erfindungsgemäß weist das Mittel zur Reinigung der Haut ein organisches Lösemittel auf, das eine geringere Flüchtigkeit als Ethanol und einen Siedepunkt > 78,32 °C hat,
wobei der Siedepunkt bei einem Druck von 1,013 bar gemessen wird. Vorzugsweise ist das Lösemittel mindestens ein
- mehrwertiger Alkohol oder ein Derivat eines mehrwertigen Alkohols, insbesondere ein Polydiol, vorzugsweise ein Alkylenglykol, ein Polyalkylenglykol, ein Polydiolderivat, vorzugsweise ein Polyalkylenglykolether und/oder ein Polyalkylenglykolester,
- Mono- oder Polyester einer gesättigten oder ungesättigten ein- oder mehrwertigen Carbonsäure mit 2 bis 30 Kohlenstoffatomen mit n- und iso-Alkanolen mit 2 bis 10 Kohlenstoffatomen, vorzugsweise ein Diester von aliphatischen und/oder aromatischen Di- und/oder Tricarbonsäuren und/oder
- aliphatischer Kohlenwasserstoff mit C₁₂- bis C₂₂- Kohlenstoffatomen, vorzugsweise mit C₁₆- bis C₂₀- Kohlenstoffatomen und besonders bevorzugt Isohexadecan,
oder eine Mischung aus mindestens zwei der oben genannten Substanzen.

Bevorzugte Polyalkylenglykole oder deren Ester oder Ether sind in den Tabellen aus Ullmann 4. Auflage, Band 8, Seiten 200, 204, 205, 207 und Band 19, Seiten 426 und 428 aufgelistet.

Besonders bevorzugt ist das Lösemittel Dipropylenglykolmonomethylether.

In einer bevorzugten Ausführungsform der vorliegenden Erfindung ist das Polymere in der wasserbasierenden Polyisoprenlatexemulsion Polyisopren, das einen cis 1, 4 - Gehalt von mindestens 90% aufweist. Vorzugsweise hat die Polyisoprenlatexemulsion einen Feststoffgehalt von 50 bis 80, besonders bevorzugt 60 bis 70 Gew.-%. Die Partikelgröße des Polyisopren beträgt vorzugsweise maximal 1,8 µm.

Vorzugsweise beträgt das Verhältnis von der Polyisoprenlatexemulsion zum Lösemittel 8:1 bis 1:1, besonders bevorzugt 4:1 bis 2:1. Gegebenenfalls weist das erfindungsgemäße Mittel zur Reinigung der Haut ein Tensid auf. Vorzugsweise ist dieses Tensid ein Fettalkoholethoxylat der allgemeinen Formel: R-O-(CH₂-CH₂-O)ₙ mit R=C₈₋₁₈ und n=1-8, vorzugsweise R=C₁₂₋₁₄ und n=4-6. Ebenfalls bevorzugt ist das Tensid ein Fettalkoholethersulfat der allgemeinen Formel: R-O-(CH₂-CH₂-O)ₙ-SO₄X₂ mit R=C₁₀₋₁₆ und n=1-4 und X= Na⁺ K⁺, ½ Mg²⁺ oder NH₄⁺.

Vorzugsweise enthält das erfindungsgemäße Mittel zur Reinigung der Haut mindestens ein Abrasivum. Die Abrasiva können die üblichen Abrasiva oder deren Mischung, vorzugsweise gebleichtes Walnußschalenmehl, nichtquellbare Stärke oder deren Mischung sein.

Zur Beeinflussung der Konsistenz des erfindungsgemäßen Mittels zur Reinigung der Haut kann diese noch wasserquellbare Polymere als Verdickungsmittel enthalten, wobei Polymere und Copolymere erhältlich durch die Polymerisation von Acrylsäure gegebenenfalls mit anderen Acrylsäurederivaten als Comonomere bevorzugt eingesetzt werden.

Das erfindungsgemäße Mittel zur Reinigung der Haut kann kosmetische Hilfs- oder Zusatzstoffe und/oder Wirkstoffe enthalten, wie z. B. Pflegederivate, Emollients, Parfum (Duftstoffe), Konservierungsmittel u. a.

Das erfindungsgemäße Mittel zur Reinigung der Haut hat den Vorteil, daß es toxikologisch unbedenklich und für die Umwelt weniger belastend ist. Die Reinigungsleistung des erfindungsgemäßen Mittels zur Reinigung der Haut entspricht denen des Standes der Technik. Das erfindungsgemäße Mittel zur Reinigung der Haut kann in Lackierbetrieben eingesetzt werden.

Das erfindungsgemäße Mittel zur Reinigung der Haut eignet sich insbesondere als Waschpaste. Gegenstand der vorliegenden Erfindung ist deshalb auch die Verwendung des erfindungsgemäßen Mittels zur Reinigung der Haut als Waschpaste, insbesondere als Handwaschpaste.

Die erfindungsgemäße Verwendung hat den Vorteil, daß sie toxikologisch unbedenklich ist und die Umwelt weniger belastet. Die Reinigungsleistung des erfindungsgemäßen Mittels zur Reinigung der Haut entspricht denen des Standes der Technik. Die erfindungsgemäße Verwendung kann vorteilhaft in Lackierbetrieben erfolgen.

Ein weiterer Gegenstand der vorliegenden Erfindung ist ein Verfahren zum Reinigen der Haut, bei dem das erfindungsgemäße Mittel auf die Haut aufgetragen und verrieben wird, um Partikel auszubilden, die bei weiterem Reiben von der Haut abfallen. Das Reiben dauert, z. B. beim Reinigen der Hände 10 bis 60 Sekunden, vorzugsweise 10 bis 30 Sekunden.

Das erfindungsgemäße Verfahren hat den Vorteil, daß es toxikologisch unbedenklich ist und die Umwelt weniger belastet. Die Reinigungsleistung des erfindungsgemäßen Mittels zur Reinigung der Haut entspricht denen des Standes der Technik. Das erfindungsgemäße Verfahren kann in Montagebetrieben und Werkstätten sowie auch in Lackierbetrieben eingesetzt werden, wobei Benetzungsstörungen auf Werkstoffoberflächen weitgehend vermieden werden.

Im folgenden wird die Erfindung anhand von Beispielen erläutert. Diese Erläuterungen sind lediglich beispielhaft.

### Prüfmethoden:

### 1. Hautverträglichkeit mit Hilfe des Duhring-Kammer-Testes:

Bei dieser Methode handelt es sich um ein in vivo-Testmodell zur Überprüfung der Hautverträglichkeit verschiedener Testprodukte in direktem Vergleich. Den 20 Probanden werden die zu testenden Produkte in luftdurchlässigen Aluminiumkammern (Finn-Chambers^{®} 18 mm Durchmesser) auf die volare Seite der Unterarme an vier Tagen hintereinander auf jeweils dasselbe Testareal appliziert. Die Applikationszeiten betragen zwei Stunden am ersten, vier Stunden am zweiten und jeweils sechs Stunden am dritten und vierten Tag. Die Befestigung der Finn-Chamber^{®} erfolgt mit Pflasterstreifen. Bei starken dermalen Effekten wird der Test für das jeweilige Testfeld auch vor erreichen der Gesamtapplikationszeit beendet.

Beurteilt werden die entstandenen Hautirritationen nach der unten angegebenen Skala bzw. die Applikationszeiten.
R= Röttung (Erythem): 0= kein Erythem 4= starkes Erythem
S= Schuppung: 0= keine Schuppung 4= starke Schuppung
F=Fissuren: 0= keine Fissuren 4= starke Fissuren

Als Beurteilungskriterien ergeben sich die:
a. Irritaton als Mittelwert der Summe der Irritationswerte von R, S und F von n Probanden
b. Applikationszeit als Mittelwert der tolerierten Applikationszeiten in Stunden von n Probanden.

### 2. Prüfung der Reinigungsleistung

Mit mindestens acht Probanden werden zwei Produkte vergleichend geprüft. Voraussetzung ist, daß alle Probanden eine charakteristische, durch manuelle Arbeit bedingte Hautstruktur der Handinnenflächen besitzen. Morgens und nachmittags wird mit je einem Produkt folgender Test durchgeführt:
- eine definierte Menge Modellschmutz (0,2 bis 0,5 g) wird auf die Handinnenfläche und auf den Handrücken verteilt und 45 s verrieben
- 1 ½ Min. trocknen lassen
- eine definierte Menge des Testproduktes (0,3 bis 1,8 g) wird aufgetragen und eingerieben
- es wird solange gerieben, bis nach Antrocknen das Produkt mit dem gebundenen Schmutz unter Reiben von der Haut abgetrennt (abgeribbelt) und entfernt ist,
- visuelle Beurteilung der Restverschmutzung (RV) auf dem Handrücken und der Handinnenfläche gemäß Skala s.u.
0 = sauber 5 = kein Reinigungseffekt (Abstufung in 0,5er Schritten möglich)
RVᵢₙₙₑₙ = Mittelwert der Restverschmutzung Handinnenflächen von n Meßreihen (Probanden)
RV_{außen} = Mittelwert der Restverschmutzung Handaußenflächen von n Meßreihen (Probanden)

Zusammenfassung eines für die Prüfung geeigneten Modellschmutzes:
Motoröl 54,15 %
Vaseline 18,05 %
Adeps lanae 18,05 %
Graphit 3,61 %
Flammruß 5,42 %
Eisenoxid (Fe₂O₃) 0,72 %

### 3. Toxikologische Prüfung

Die toxikologische Wirkung der natürlichen und/oder synthetischen wasserbasierenden Polyisoprenlatexemulsion kann durch die Bestimmung der Zytotoxizität und den Neutralrot-Test festgestellt werden.

Die Durchführung des Tests erfolgt nach der EURO-Norm EN 30 993-5, wobei unter Auslassung der 72h-Inkubationszeit die Prüfsubstanz in Zellkulturmedium verdünnt auf die Zellen appliziert werden.

Als Latexemulsion wurden ein Polyisopren-Latex mit der Bezeichnung KRATON^{®} IR-RP 401 der Shell AG eingesetzt.

| | | | |
|---|---|---|---|
| Zellen | : Balb/c 3T3 | Vehikel | : DMEM |
| Verdünnung | : 10% (w/v) | pH-Wert | : 8,22 |

### Neutralrot-Aufnahme:

| Konzentration (mg/ml) der Prüfsubstanz | 0 | 1 | 2,5 | 5 | 7,5 | 10 | 25 | 50 | 100 |
|---|---|---|---|---|---|---|---|---|---|
| Hemmung (%) der Neutralrot-Aufnahme | | -5,89 | -8,70 | 15,07 | 38,44 | 57,74 | 72,22 | 80,31 | 82,42 |

| | |
|---|---|
| NR₅₀-Wert: | VV: 7,0 mg/ml (Vorversuch) |
| 2. | V: 9,0 mg/ml (Versuch) |

### Bewertung:

Aus diesen Untersuchungen ergibt sich ein NR₅₀-Wert von 7,0 mg/ml bzw. 9,0 mg/ml. Das Produkt ist daher als schwach zytotoxisch einzustufen.

### Beispiele

Zur Prüfung der Reinigungsleistung und der Hautverträglichkeit wurden 9 Handwaschpasten hergestellt, deren Zusammsensetzung der folgenden Tabelle 1 entnommen werden kann, wobei die Angaben in Gew.-% gemacht sind.

**Tabelle 1**

| Beispiel | 1 | 2 | 3 | 5 | 6 | 7 | 8 | 9 |
|---|---|---|---|---|---|---|---|---|
| Kraton IR 401 Latex | 17 | 30 | 50 | 80 | 80 | 30 | 30 | 30 |
| PPG-2-Methylether | 15 | 10 | 5 | 10 | 5 | | | |
| Di-n-butyladipat | | | | | | 10 | | |
| PEG 4 | | | | | | | 10 | |
| Hexylenglycol | | | | | | | | 10 |
| Isohexadecan | | | | | | | | |
| Pareth-5 | | 3 | 3 | 3 | | 3 | 3 | 3 |
| Laureth-6 | 1 | | | | 3 | | | |
| Wasser | 60 | 50 | 35 | 0 | 5 | 50 | 50 | 50 |
| Walnußschalenmehl | 6 | 6 | 6 | 6 | 6 | 6 | 6 | 6 |
| Verdicker | 1 | 1 | 1 | 1 | 1 | 1 | 1 | 1 |
| Gesamt | 100 | 100 | 100 | 100 | 100 | 100 | 100 | 100 |

**Tabelle 2**

| Hautverträglichkeit | 0 | 0 | + | 0 | + | + | + | + |
|---|---|---|---|---|---|---|---|---|
| Hautreinigung im Vergleich zu Castrol Super Clean | + | + | 0 | + | 0 | + | 0 | 0 |

| | | | | | | | | |
|---|---|---|---|---|---|---|---|---|
| Kraton^{™} IR 401 Latex = Isopren-Latex der Firma Shell Chemicals UT Ltd. PPG-2-Methylether = Dipropylenglykolmonomethylether PEG 4= Polyethylenglykol 400 P Pareth-5 = Polyethylenglykolether eines Gemisches aus synthetischem C₉-C₁₁-Fettalkohol (n = 5) Laureth-6 = Laurylalkoholpolyethylenglykolether (n = 6) Verdicker = Carbopol ETD 2020: Acrylsäure-Copolymer (98 - 100%) | | | | | | | | |

Alle in der Tabelle 1 angegebenen Substanzen wurden toxikologisch bewertet und als unproblematisch eingestuft. Alle beispielhaften Waschpasten können in Lakierbetrieben eingesetzt werden.

Die Handwaschpasten gemäß den Beispielen 1-9 wurden mit Castrol Super Clean einem Produkt der Castrol Ltd. verglichen. Die Ergebnisse des Vergleiches sind in Tabelle 2 zusammengefaßt, wobei 0 vergleichbar und + besser bedeutet.

## Patentansprüche

1. Mittel zur Reinigung der Haut, welches dazu geeignet ist, auf der Haut verrieben zu werden, um Partikel auszubilden, die bei weiterem Reiben von der Haut abfallen, und die Haut ohne Zusatz von Wasser zu reinigen, enthaltend
- eine flüssige natürliche und/oder synthetische wasserbasierende Polyisoprenlatexemulsion mit einem Durchmesser der Polymerpartikel von 0,05 bis 5 µm,
- ausschließlich mindestens ein organisches Lösemittel, das eine geringere Flüchtigkeit als Ethanol und einen Siedepunkt > 78,32 °C aufweist, und
- Wasser.

2. Mittel zur Reinigung der Haut nach Anspruch 1 enthaltend:
a) 10-80 Gew.-% einer flüssigen natürlichen und/oder synthetischen wasserbasierenden Polyisoprenlatexemulsion,
b) 1-15 Gew.-% eines organischen Lösemittels, das eine geringere Flüchtigkeit als Ethanol und einen Siedepunkt > 78,32 °C aufweist,
c) 0-10 Gew.-% mindestens eines Tensides, vorzugsweise mindestens eines Fettalkoholethoxylates, Fettalkoholethersulfates, Succinates, Sarkosides und/oder Glucosides,
d) 0-10 Gew.-% Abrasiva, vorzugsweise gebleichtes Walnussschalenmehl und/ oder nicht quellbare Stärke,
e) 0-0,1 Gew.-% Verdickungsmittel,
f) ggf. kosmetische Hilfs- und Zusatzstoffe und/oder Wirkstoffe,
g) 10-60 Gew.-% Wasser
wobei die Summe immer 100 Gew.-% ergibt.

3. Mittel zur Reinigung der Haut nach Anspruch 1 oder 2, **dadurch gekennzeichnet, dass** das Lösemittel mindestens ein
- mehrwertiger Alkohol oder ein Derivat eines mehrwertigen Alkohols, insbesondere ein Polydiol, vorzugsweise ein Alkylenglykol, ein Polyalkylenglykol, ein Polydiolderivat, vorzugsweise ein Polyalkylenglykolether und/oder ein Polyalkylenglykolester,
- ein Mono- oder Polyester einer gesättigten oder ungesättigten ein- oder mehrwertigen Carbonsäure mit 2 bis 30 Kohlenstoffatomen mit n- und iso-Alkanolen mit 2 bis 10 Kohlenstoffatomen, vorzugsweise ein Diester von aliphatischen und/oder aromatischen Di- und/oder Tricarbonsäuren und/oder
- ein aliphatischer Kohlenwasserstoff mit C₁₂- bis C₂₂ Kohlenstoffatomen, vorzugsweise mit C₁₆- bis C₂₀- Kohlenstoffatomen und besonders bevorzugt Isohexadecan,
ist.

4. Mittel zur Reinigung der Haut nach einem der Ansprüche 1 bis 3, **dadurch gekennzeichnet, dass** das Lösemittel Dipropylenglykolmonomethylether ist.

5. Mittel zur Reinigung der Haut nach einem der Ansprüche 1 bis 4, **dadurch gekennzeichnet, dass** der Feststoffgehalt der Polyisoprenlatexemulsion 50 bis 80, vorzugsweise 60 bis 70 Gew.-%, beträgt.

6. Mittel zur Reinigung der Haut nach einem der Ansprüche 1 bis 5, **dadurch gekennzeichnet, dass** die Polyisoprenlatexemulsion einen cis 1,4-Gehalt von mindestens 90% hat.

7. Mittel zur Reinigung der Haut nach einem der Ansprüche 1 bis 6, **dadurch gekennzeichnet, dass** die Partikelgröße im Polyisoprenlatex maximal 1,8 µm beträgt.

8. Mittel zur Reinigung der Haut nach einem der Ansprüche 2 bis 7, **dadurch gekennzeichnet, dass** das Tensid ein Fettalkoholethoxylat der allgemeinen Formel: R-O-(CH₂-CH₂-O)ₙ mit R=C₈₋₁₈ und n=1-8, vorzugsweise R=C₁₂₋₁₄ und n=4-6 ist.

9. Mittel zur Reinigung der Haut nach einem der Ansprüche 2 bis 7, **dadurch gekennzeichnet, dass** das Tensid ein Fettalkoholethersulfat der allgemeinen Formel: R-O-(CH₂-CH₂-O)ₙ-SO₄X₂ mit R=C₁₀₋₁₆ und n=1-4 und X= Na⁺K⁺, ½ Mg²⁺ oder NH₄⁺ ist.

10. Verwendung des Mittels nach einem der Ansprüche 1 bis 9 als Waschpaste, insbesondere als Handwaschpaste.

11. Verfahren zum Reinigen der Haut, **dadurch gekennzeichnet, dass** das Mittel nach einem der Ansprüche 1 bis 9 auf die Haut aufgetragen und verrieben wird, um Partikel auszubilden, die bei weiterem Reiben von der Haut abfallen, wobei die Reinigung ohne Zusatz von Wasser erfolgt.

## Claims

1. A skin-cleansing agent adapted to be rubbed onto the skin to form particles which on continued rubbing drop off the skin, and adapted to cleanse the skin without the addition of water, containing
• a liquid natural and/or synthetic water-based polyisoprene latex emulsion in which the diameter of the polymer particles ranges from 0.05 to 5 µm,
• exclusively at least one organic solvent showing less volatility than ethanol and having a boiling point above 78.32 °C, and
• water.

2. The skin-cleansing agent as defined in claim 1, containing:
a) from 10 to 80 wt% of a liquid natural and/or synthetic water-based polyisoprene latex emulsion,
b) from 1 to 15 wt% of an organic solvent showing less volatility than ethanol and having a boiling point above 78.32 °C,
c) from 0 to 10 wt% of at least one surfactant, preferably at least one fatty alcohol ethoxylate, fatty alcohol ether sulfate, succinate, sarcoside, and/or glucoside,
d) from 0 to 10 wt% of abrasives, preferably bleached walnut shell flour and/or non-swellable starch,
e) from 0 to 0.1 wt% of thickener,
f) optionally cosmetic auxiliaries and additives and/or active ingredients, and
g) from 10 to 60 wt% of water,
always totalling 100 wt%.

3. The skin-cleansing agent as defined in claim 1 or claim 2, **characterized in that** said solvent is
• at least one polyhydric alcohol or a derivative of a polyhydric alcohol, in particular a polydiol, preferably an alkylene glycol, a polyalkylene glycol, a polydiol derivative, preferably a polyalkylene glycol ether and/or a polyalkylene glycol ester,
• at least one monoester or polyester of a saturated or unsaturated monovalent or polyvalent carboxylic acid containing from 2 to 30 carbons with n-alkanols and isoalkanols containing from 2 to 10 carbons, preferably a diester of aliphatic and/or aromatic dicarboxylic acids and/or tricarboxylic acids, and/or
• at least one aliphatic hydrocarbon containing from 12 to 22 carbons, preferably from 16 to 20 carbons, and more preferably isohexadecane.

4. The skin-cleansing agent as defined in any one of claims 1 to 3, **characterized in that** said solvent is dipropylene glycol monomethylether.

5. The skin-cleansing agent as defined in any one of claims 1 to 4, **characterized in that** the solids content of said polyisoprene latex emulsion is from 50 to 80 wt% and preferably from 60 to 70 wt%.

6. The skin-cleansing agent as defined in any one of claims 1 to 5, **characterized in that** said polyisoprene latex emulsion has a cis-1,4 content of at least 90 %.

7. The skin-cleansing agent as defined in any one of claims 1 to 6, **characterized in that** the particle size in the polyisoprene latex is not more than 1.8 µm.

8. The skin-cleansing agent as defined in any one of claims 2 to 7, **characterized in that** said surfactant is a fatty alcohol ethoxylate of the general formula: R-O-(CH₂-CH₂-O)ₙ, in which R denotes C₈₋₁₈ and n equals 1 - 8 and preferably R denotes C₁₂₋₁₄ and n equals 4 - 6.

9. The skin-cleansing agent as defined in any one of claims 2 to 7, **characterized in that** said surfactant is a fatty alcohol ether sulfate of the general formula: R-O-(CH₂-CH₂-O)ₙ-SO₄X₂, in which R denotes C₁₀₋₁₆ and n equals 1 - 4 and X denotes Na⁺K⁺, ½ Mg²⁺, or NH₄⁺.

10. The use of the agent as defined in any one of claims 1 to 9 as a washing paste, particularly a hand washing paste.

11. A method of cleansing the skin, **characterized in that** the agent as defined in any one of claims 1 to 9 is applied to the skin and rubbed so as to form particles which on continued rubbing drop off the skin, such cleansing being carried out without the addition of water.

## Revendications

1. Agent pour nettoyer la peau qui convient pour être frotté sur la peau, pour former des particules, qui tombent de la peau lorsqu'on continue à frotter et qui nettoient la peau sans addition d'eau, contenant
- une émulsion de latex de polyisoprène liquide, naturelle et/ou synthétique à base d'eau, avec un diamètre des particules polymères de 0,05 à 5 µm,
- exclusivement au moins un solvant organique, qui présente une volatilité plus basse que l'éthanol et un point d'ébullition > 78,32°C, et
- de l'eau

2. Agent pour nettoyer la peau selon la revendication 1, contenant :
a) 10-80% en poids d'une émulsion de latex de polyisoprène liquide, naturelle et/ou synthétique à base d'eau,
b) 1-15% en poids d'un solvant organique, qui présente une volatilité plus basse que l'éthanol et un point d'ébullition > 78,32°C,
c) 0-10% en poids d'au moins un agent tensioactif, de préférence d'au moins un éthoxylate d'alcool gras, d'un éthersulfate d'alcool gras, d'un succinate, d'un sarcoside et/ou d'un glucoside,
d) 0-10% en poids d'agents abrasifs, de préférence de la farine de coquille de noix blanchie et/ou de l'amidon non gonflable,
e) 0-0,1% en poids d'un épaississant,
f) le cas échéant des adjuvants, des additifs et/ou des substances actives cosmétiques,
g) 10-60% en poids d'eau
la somme étant toujours égale à 100% en poids.

3. Agent pour nettoyer la peau selon la revendication 1 ou 2, **caractérisé en ce que** le solvant est au moins
- un alcool polyvalent ou un dérivé d'un alcool polyvalent, en particulier un polydiol, de préférence un alkylèneglycol, un polyalkylèneglycol, un dérivé de polydiol, de préférence un polyalkylèneglycoléther et/ou un ester de polyalkylèneglycol,
- un monoester ou un polyester d'un acide carboxylique saturé ou insaturé, monovalent ou polyvalent, comprenant 2 à 30 atomes de carbone avec des n-alcanols et des iso-alcanols comprenant 2 à 10 atomes de carbone, de préférence un diester d'acides dicarboxyliques et/ou tricarboxyliques aliphatiques et/ou aromatiques et/ou
- un hydrocarbure aliphatique comprenant 12 à 22 atomes de carbone, de préférence 16 à 20 atomes de carbone et de manière particulièrement préférée de l'isohexadécane.

4. Agent pour nettoyer la peau selon l'une quelconque des revendications 1 à 3, **caractérisé en ce que** le solvant est du dipropylèneglycolmonométhyléther.

5. Agent pour nettoyer la peau selon l'une quelconque des revendications 1 à 4, **caractérisé en ce que** la teneur en solides de l'émulsion de latex de polyisoprène est de 50 à 80, de préférence de 60 à 70% en poids.

6. Agent pour nettoyer la peau selon l'une quelconque des revendications 1 à 5, **caractérisé en ce que** l'émulsion de latex de polyisoprène présente une teneur en cis-1,4 d'au moins 90% en poids.

7. Agent pour nettoyer la peau selon l'une quelconque des revendications 1 à 6, **caractérisé en ce que** la grosseur des particules dans le latex de polyisoprène est d'au maximum 1,8 µm.

8. Agent pour nettoyer la peau selon l'une quelconque des revendications 2 à 7, **caractérisé en ce que** l'agent tensioactif est un éthoxylate d'alcool gras de formule générale : R-O-(CH₂-CH₂-O)ₙ avec R = C₈₋₁₈ et n = 1-8, de préférence R = C₁₂₋₁₄ et n = 4-6.

9. Agent pour nettoyer la peau selon l'une quelconque des revendications 2 à 7, **caractérisé en ce que** l'agent tensioactif est un éthersulfate d'alcool gras de formule générale : R-O-(CH₂-CH₂-O)ₙ-SO₄X₂ avec R = C₁₀₋₁₆ et n = 1-4, et X = Na⁺K⁺, 1/2 Mg²⁺ ou NH₄⁺.

10. Utilisation de l'agent selon l'une quelconque des revendications 1 à 9 comme pâte de lavage, en particulier comme pâte de lavage des mains.

11. Procédé pour nettoyer la peau, **caractérisé en ce que** l'agent selon l'une quelconque des revendications 1 à 9 est appliqué sur la peau et est frotté, pour former des particules qui tombent de la peau lorsqu'on continue à frotter, le nettoyage se réalisant sans addition d'eau.
